# EUROPEAN PATENT APPLICATION

(11) **EP 3 372 592 A1**
(43) Date of publication of application: **12.09.2018**
(21) Application number: 18160097.4
(22) Date of filing: 06.03.2018
(51) Int. Cl.: C07D 249/12, A61K 31/4196, A61P 9/00, A61P 13/12, A61P 7/00

(54) **SOLID FORMS OF LESINURAD AMINE SALTS**

(30) Priority: 07.03.2017 CZ 20170125; 21.11.2017 CZ 20170748
(71) Applicant: Zentiva, k.s., Dolni Mecholupy 102 37 Praha 10 (CZ)
(72) Inventor: Zvatora, Pavel, 19800 Praha 9 (CZ); Dammer, Ondrej, 25301 Hostivice (CZ); Krejcik, Lukas, 19017 Praha 9 (CZ); Schongut, Marek, 14000 Praha 4 (CZ); Stach, Jan, 10400 Praha 10 (CZ); Halama, Ales, 53009 Pardubice (CZ); Kutzendorferova, Petra, 10900 Praha 9 (CZ); Benediktova, Kristyna, 14900 Praha 4 (CZ); Man, Stanislav, 68732 Nezdenice (CZ); Tkadlecova, Marcela, 16300 Praha 6 (CZ)
(74) Representative: Hartvichova, Katerina

(57) **Abstract**

The invention provides solid forms of lesinurad with an amino group-containing organic base of formula

R¹-NH-R²,

wherein the R¹ and R² groups are independently selected from the group consisting of -C(=O)-NH₂, hydrogen, a linear or branched C1-C6 alkyl or C1-C6 cycloalkyl while the alkyl or cycloalkyl are optionally substituted by at least one hydroxy group,
provided that at least one of the R¹ and R² groups is not hydrogen.

Further, an amorphous and crystalline form of lesinurad free acid are disclosed.

Methods of production and medical uses of the solid forms of lesinurad are also provided by the present invention.

## Description

### Field of the Invention

The invention relates to solid forms of 2-(5-bromo-4-(1-cyclopropylnaphtalen-4-yl)-4*H*-1,2,4-triazol-3-ylthio)acetic acid of formula 1, 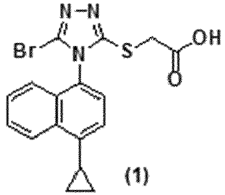 known under the generic name lesinurad, methods of production and uses thereof.

### Background Art

Lesinurad, systematically 2-(5-bromo-4-(1-cyclopropylnaphtalen-4-yl)-4*H*-1,2,4-triazol-3 - ylthio)acetic acid, is a selective inhibitor of reabsorption of uric acid, which inhibits the uric acid transporter URAT1, thus reducing the level of uric acid in blood. It is used in combination with some of the drugs acting as xanthine oxidase inhibitors in patients where a satisfactory condition is not achieved with xanthine oxidase inhibitors alone.
A drug containing lesinurad is currently approved by FDA (U. S. Food and Drug Administration) and is commercially available under the trade name Zurampic from AstraZeneca. This preparation contains a dose of 200 mg of free acid in one tablet and is intended for the treatment of hyperuricemia, being co-administered with xanthine oxidase inhibitors (allopurinol or febuxostat). The approved daily dose is maximum 200 mg for one month.
The molecule of lesinurad was first described in the patent application WO 2006/026356. An alternative synthesis of this active ingredient was described in WO 2012/092395 and WO 2014/008295.

Active pharmaceutical ingredients frequently exist in various crystalline forms, called polymorphs, or they can create hydrates, solvates, salts or cocrystals with other compounds. Individual solid phases differ from each other in arrangement of molecules, which gives them different physicochemical properties such as melting point, stability, solubility or dissolution rate. To distinguish them from each other, several solid-state analytical methods can be used, e.g. X-ray powder diffraction, solid-state NMR, Raman spectroscopy as well as thermoanalytical methods. Lesinurad can exist either in the free acid form or in the form of salts as well as cocrystals. Solid forms with metals, strong acids and several organic molecules piperazine, D-glucitol, L-arginine, trichloromethane, 2-methyltetrahydrofuran and 1,5-dihydro-4*H*-pyrazolo[3,4-*d*]pyrimidin-4-one are known in the art.

In the commercially available drug sold under the trade name Zurampic, lesinurad is used in the form of free acid. It exists in several crystalline forms out of which form 2 is the most thermodynamically stable one. An assessment report of the European Medicines Agency of the original preparation Zurampic describes that the product contains a number of minor impurities and the toxicology of those that did not meet the limits imposed by the ICH Q3A Directive had to be checked. A disadvantage of the form 2 is its poor solubility which inhibits releasing of lesinurad from the drug form in the patient's body, reducing its bioavailability.

So far, several solid forms of lesinurad have been described. Although preparation of a salt by a reaction of an acid and base is a well-known method, it is always a problem to obtain the required salts in the solid phase and purity corresponding to the demands for their pharmaceutical use. Thus, there is an obvious need for novel solid forms of lesinurad in the art that will have optimum combination of characteristics necessary for the preparation of a drug form, especially from the point of view of thermodynamic stability and bioavailability of the active ingredient, better physico-chemical properties for use in pharmacy and in formulation of novel drug forms, and forms that can be easily purified. The present invention introduces several advantageous and industrially applicable crystalline solid forms of lesinurad in the form of pharmaceutically acceptable salts that have not been described so far and that exhibit high purity and can be used in a drug form at the same time.

### Disclosure of the Invention

The present invention relates to solid forms of lesinurad with amino group-containing organic bases.
In the context of the present invention, the term amino group-containing organic base refers to a primary or secondary amine or amide of formula

R¹-NH-R²,

wherein the R¹ and R² groups are independently selected from the group consisting of -C(=O)-NH₂, hydrogen, a linear or branched C1-C6 alkyl or C1-C6 cycloalkyl wherein the alkyl or cycloalkyl are optionally substituted by at least one hydroxy group,
provided that at least one of the R¹ and R² groups is not hydrogen.

Preferably, R¹ is hydrogen or methyl and R² is -C(=O)-NH₂.

In a preferred embodiment, the R¹ and R² groups independently stand for hydrogen or a linear or branched C1-C6 alkyl or C1-C6 cycloalkyl, while the alkyl or cycloalkyl are substituted by at least one hydroxy group, provided that at least one of the R¹ and R² groups is not hydrogen.

The compounds bearing a linear or branched C1-C6 alkyl or C1-C6 cycloalkyl wherein the alkyl or cycloalkyl are optionally substituted by at least one hydroxy group, are herein called alkylamines.

Preferably, the alkylamine is selected from tromethamine, diethylamine, cyclohexylamine, or tert-butylamine. Preferably, the alkylamine is not isopropylamine.

In a preferred embodiment, the alkylamine is tromethamine, also known as tris(hydroxymethyl)aminomethane. Most preferably, the solid form of lesinurad with tromethamine is a form II solid form, as described herein below.

In the context of the present invention, the term solid form of lesinurad with an alkylamine refers to a salt or cocrystal of lesinurad with the amino group-containing organic base in a solid form. The two constituents can be present preferably in a molar ratio within the range from 2:1 to 1:2, more preferably in a molar ratio within the range from 1.5:1 to 1:1.5, most preferably in a molar ratio 1:1. In particular when the solid form is a salt, the amino group-containing organic base may be in cationic form.

Solid forms of lesinurad with amino group-containing organic bases, such as urea, tromethamine or other alkylamines, can be prepared in high yields with high chemical purity in crystalline form, amorphous form, or in a mixture of a crystalline and amorphous solid form. Preferably, the solid forms of lesinurad are in crystalline form.

The solid forms of lesinurad with alkylamines according to the invention make it possible to prepare lesinurad in a solid crystalline form with higher solubility and bioavailability than in the case of lesinurad free acid. While the dissolution rate of crystalline form 2 of lesinurad free acid prepared according to the procedure disclosed in the patent application WO 2012/092395 was 0.42 mg/min/cm², the dissolution rate of lesinurad with tromethamine, measured under identical conditions was 7.44 mg/min/cm² and the dissolution rate of lesinurad with diethylamine was 21.58 mg/min/cm². Thus, the solid form of lesinurad with tromethamine was about 17 times more soluble that crystalline form 2 of lesinurad free acid. Considering the salt with diethylamine, the difference in solubility was almost 50 times.

Though higher solubility is usually accompanied by lower thermodynamic stability, the prepared solid forms of lesinurad with alkylamines according to the invention surprisingly exhibit comparable or even slightly better thermodynamic stability than lesinurad free acid. The melting point of crystalline form 2 of lesinurad free acid, prepared according to the procedure disclosed in the patent application WO 2012/092395 was determined to be 172°C with the use of Differential Scanning Calorimetry (DSC). The melting point of the individual solid forms of lesinurad with tromethamine was determined to be 169°C, or 177°C, respectively, using the same method. Thus, these are comparable solid forms from the thermodynamic stability point of view.

The prepared solid forms of lesinurad may have various internal arrangements (polymorphism) with different physico-chemical properties depending on the conditions of their preparation. For this reason, the invention relates to individual crystalline forms as well as to their mixtures.

In one embodiment of the invention, lesinurad is in the form of a solid form with tromethamine.

The solid form of lesinurad with tromethamine preferably shows at least one of the following properties: a) characteristic reflections in the X-ray powder spectrum: 10.5; 14.1; 17.4; 21.6; 23.9 and 25.7 ± 0.2° 2-theta, and/or b) a peak in the DSC (differential scanning calorimetry record) at the temperature of 169°C (referred to as form I).

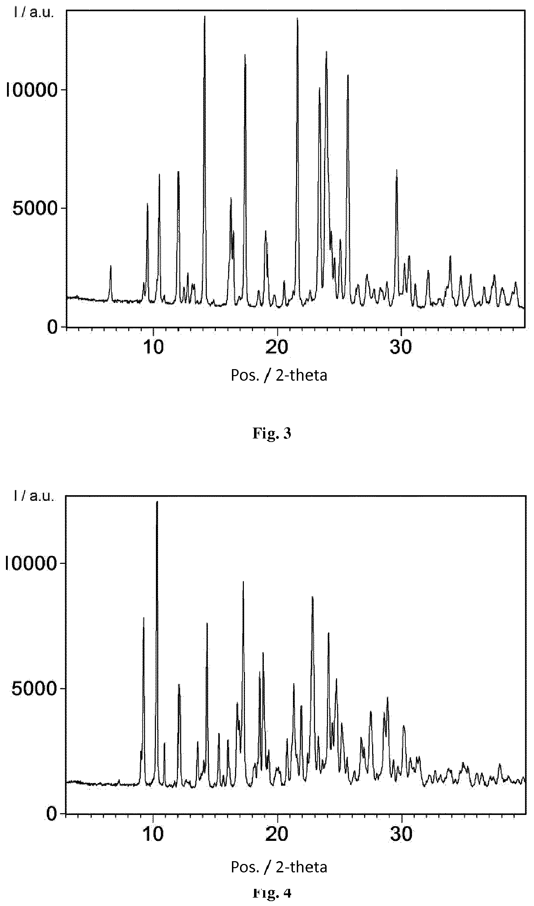

The crystalline form of lesinurad with tromethamine (form I - prepared according to Example 3) is characterized by the reflections presented in Table 1. Table 1 includes reflections whose relative intensity value is higher than 1%. Characteristic diffraction peaks of the crystalline form I of lesinurad with tromethamine according to the present invention with the use of CuKα radiation are: 10.5; 14.1; 17.4; 21.6; 23.9 and 25.7 ± 0.2° 2-theta. The X-ray powder pattern is shown in Fig. 3.

**Table 1**

| Position [°2Th.] | Interplanar spacing [Å] [Å]=0.1nm | Rel. intensity [%] |
|---|---|---|
| 6.52 | 13.543 | 11.4 |
| 9.49 | 9.313 | 32.0 |
| 10.46 | 8.453 | 43.2 |
| 12.00 | 7.371 | 44.4 |
| 12.73 | 6.946 | 10.3 |
| 14.10 | 6.278 | 96.5 |
| 16.22 | 5.461 | 36.3 |
| 17.37 | 5.100 | 85.7 |
| 19.07 | 4.651 | 25.2 |
| 20.52 | 4.325 | 8.7 |
| 21.60 | 4.111 | 100.0 |
| 23.37 | 3.804 | 75.5 |
| 23.92 | 3.717 | 84.9 |
| 24.32 | 3.657 | 26.1 |
| 24.59 | 3.618 | 16.3 |
| 25.05 | 3.552 | 23.0 |
| 25.67 | 3.467 | 79.6 |
| 26.43 | 3.369 | 6.6 |
| 27.14 | 3.283 | 7.3 |
| 27.75 | 3.212 | 6.1 |
| 28.81 | 3.097 | 7.9 |
| 29.61 | 3.015 | 49.6 |
| 30.23 | 2.954 | 15.2 |
| 30.60 | 2.919 | 16.7 |
| 31.11 | 2.873 | 8.6 |
| 32.14 | 2.782 | 12.7 |
| 33.90 | 2.642 | 18.9 |
| 34.74 | 2.580 | 10.1 |
| 35.55 | 2.523 | 11.6 |
| 36.65 | 2.450 | 7.3 |
| 37.47 | 2.398 | 10.8 |

Differential scanning calorimetry (DSC) was applied to measure the melting point of the crystalline form of lesinurad with tromethamine (form I) of 169°C.

In another preferred embodiment, the solid form of lesinurad with tromethamine has at least one of the following properties: a) characteristic reflections in the X-ray powder pattern: 9.2; 10.3; 14.3; 17.2; 18.9; 22.8 and 24.1 ± 0.2° 2-theta, and/or b) a peak in the DSC record at the temperature of 177°C (referred to as form II).

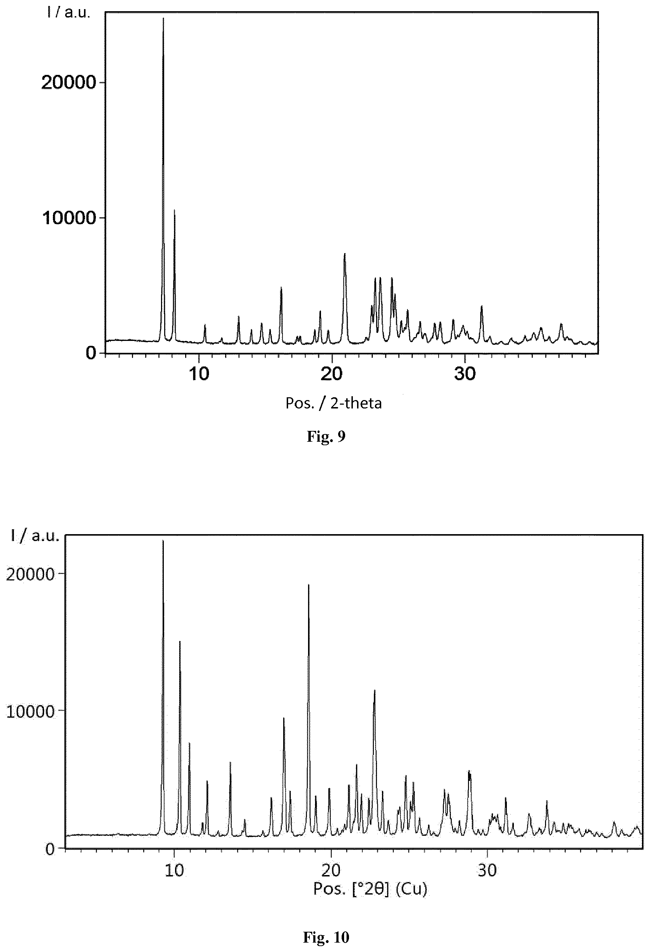

The crystalline form of lesinurad with tromethamine (form II - prepared according to Example 4) is characterized by the reflections presented in Table 2. Table 2 includes reflections whose relative intensity value is higher than 1%. Characteristic diffraction peaks of the crystalline form II of lesinurad with tromethamine according to the present invention with the use of CuKα radiation are: 9.2; 10.3; 14.3; 17.2; 18.9; 22.8 and 24.1 ± 0.2° 2-theta. The X-ray powder pattern is shown in Fig. 4 and 10.

**Table 2**

| Position [°2Th.] | Interplanar spacing [Å] [Å]=0.1nm | Rel. intensity [%] |
|---|---|---|
| 9.24 | 9.567 | 54.1 |
| 10.30 | 8.578 | 100.0 |
| 10.92 | 8.093 | 16.2 |
| 12.08 | 7.322 | 34.9 |
| 13.55 | 6.529 | 15.8 |
| 14.32 | 6.181 | 56.8 |
| 15.28 | 5.793 | 19.0 |
| 16.01 | 5.531 | 17.8 |
| 16.75 | 5.289 | 23.2 |
| 17.23 | 5.142 | 68.5 |
| 18.58 | 4.772 | 39.9 |
| 18.87 | 4.698 | 47.5 |
| 19.24 | 4.609 | 11.4 |
| 19.87 | 4.465 | 7.3 |
| 20.75 | 4.276 | 15.7 |
| 21.29 | 4.170 | 36.0 |
| 21.90 | 4.055 | 27.5 |
| 22.80 | 3.897 | 61.9 |
| 23.27 | 3.820 | 13.5 |
| 24.08 | 3.693 | 56.3 |
| 24.39 | 3.646 | 20.0 |
| 24.70 | 3.602 | 30.6 |
| 25.19 | 3.532 | 20.3 |
| 26.68 | 3.338 | 14.8 |
| 27.46 | 3.246 | 24.4 |
| 28.55 | 3.124 | 24.9 |
| 28.83 | 3.095 | 29.5 |
| 30.14 | 2.963 | 20.0 |
| 30.66 | 2.914 | 9.4 |
| 31.18 | 2.866 | 9.6 |
| 32.66 | 2.740 | 5.6 |
| 34.88 | 2.570 | 8.7 |
| 37.83 | 2.376 | 8.0 |

Differential scanning calorimetry (DSC) was applied to measure the melting point of the crystalline form of lesinurad with tromethamine (form II) of 177°C.

In another embodiment, lesinurad is in the form of a solid form with diethylamine.
The solid form of lesinurad with diethylamine preferably shows at least one of the following properties: a) characteristic reflections in the X-ray powder pattern: 7.3; 10.1; 15.7; 21.7; 23.0 and 25.1 ± 0.2° 2-theta, and/or b) a peak in the DSC record at the temperature of 117°C.

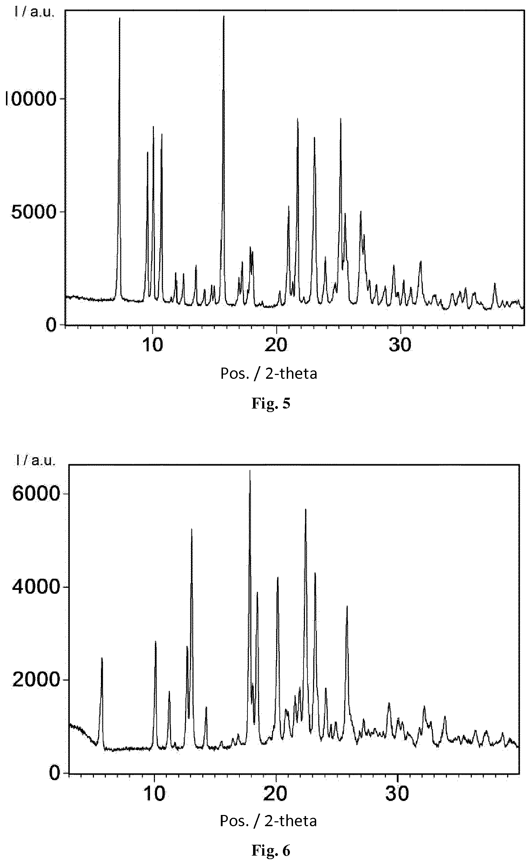

The crystalline form of lesinurad with diethylamine (prepared according to Example 5) is characterized by the reflections presented in Table 3. Table 3 includes reflections whose relative intensity value is higher than 1%. Characteristic diffraction peaks of the crystalline form of lesinurad with diethylamine according to the present invention with the use of CuKα radiation are: 7.3; 10.1; 15.7; 21.7; 23.0 and 25.1 ± 0.2° 2-theta. The X-ray powder pattern is shown in Fig. 5.

**Table 3**

| Position [°2Th.] | Interplanar spacing [Å] [Å]=0.1nm | Rel. intensity [%] |
|---|---|---|
| 7.33 | 12.046 | 86.5 |
| 9.56 | 9.244 | 48.2 |
| 10.06 | 8.786 | 57.4 |
| 10.71 | 8.253 | 52.8 |
| 11.88 | 7.443 | 11.2 |
| 12.48 | 7.089 | 10.5 |
| 13.49 | 6.558 | 14.0 |
| 14.16 | 6.250 | 5.6 |
| 14.75 | 6.001 | 7.6 |
| 15.70 | 5.640 | 100.0 |
| 16.97 | 5.221 | 10.3 |
| 17.22 | 5.146 | 15.3 |
| 17.89 | 4.953 | 20.4 |
| 18.07 | 4.907 | 19.0 |
| 20.94 | 4.239 | 34.9 |
| 21.69 | 4.095 | 61.5 |
| 23.03 | 3.858 | 56.9 |
| 23.92 | 3.717 | 18.4 |
| 25.13 | 3.541 | 67.9 |
| 25.52 | 3.488 | 31.2 |
| 26.76 | 3.329 | 36.5 |
| 27.04 | 3.295 | 26.1 |
| 28.02 | 3.182 | 8.5 |
| 28.68 | 3.110 | 6.9 |
| 29.45 | 3.031 | 15.7 |
| 30.21 | 2.956 | 10.5 |
| 31.56 | 2.833 | 16.7 |
| 34.13 | 2.625 | 6.5 |
| 34.75 | 2.580 | 6.3 |
| 35.20 | 2.548 | 7.7 |
| 37.55 | 2.393 | 9.9 |

Differential scanning calorimetry (DSC) was applied to measure the melting point of the crystalline form of lesinurad with diethylamine of 117°C.

In another embodiment, lesinurad is in the form of a solid form with tert-butylamine.

The solid form of lesinurad with tert-butylamine preferably shows at least one of the following properties: a) characteristic reflections in the X-ray powder pattern: 10.1; 13.1; 17.8; 20.1; 22.4 and 25.8 ± 0.2° 2-theta, and/or b) a peak in the DSC record at the temperature of 157°C.

The crystalline form of lesinurad with tert-butylamine (prepared according to Example 6) is characterized by the reflections presented in Table 4. Table 4 includes reflections whose relative intensity value is higher than 1%. Characteristic diffraction peaks of the crystalline form of lesinurad with tert-butylamine according to the present invention with the use of CuKα radiation are: 10.1; 13.1; 17.8; 20.1; 22.4 and 25.8 ± 0.2° 2-theta. The X-ray powder pattern is shown in Fig. 6.

**Table 4**

| Position [°2Th.] | Interplanar spacing [Å] [Å]=0.1nm | Rel. intensity [%] |
|---|---|---|
| 5.70 | 15.494 | 26.1 |
| 10.09 | 8.759 | 38.7 |
| 11.24 | 7.869 | 20.6 |
| 12.70 | 6.964 | 35.8 |
| 13.07 | 6.767 | 74.0 |
| 14.23 | 6.220 | 15.8 |
| 17.85 | 4.966 | 100.0 |
| 18.45 | 4.805 | 51.1 |
| 20.10 | 4.414 | 61.0 |
| 20.99 | 4.230 | 11.7 |
| 21.56 | 4.119 | 13.5 |
| 21.92 | 4.052 | 16.8 |
| 22.43 | 3.961 | 79.1 |
| 23.20 | 3.831 | 62.0 |
| 24.08 | 3.693 | 18.7 |
| 25.81 | 3.449 | 51.2 |
| 27.16 | 3.280 | 7.4 |
| 29.29 | 3.047 | 13.1 |
| 30.01 | 2.975 | 7.6 |
| 30.34 | 2.944 | 6.5 |
| 32.15 | 2.782 | 12.3 |
| 33.82 | 2.648 | 9.0 |
| 36.34 | 2.471 | 4.9 |
| 37.21 | 2.414 | 4.4 |
| 38.56 | 2.333 | 5.1 |

Differential scanning calorimetry (DSC) was applied to measure the melting point of the crystalline form of lesinurad with tert-butylamine of 157°C.

In another embodiment, lesinurad is in the form of a solid form with cyclohexylamine.

The solid form of lesinurad with cyclohexylamine preferably shows at least one of the following properties: a) characteristic reflections in the X-ray powder pattern: 6.7; 9.8; 13.2; 17.2; 20.2 and 23.5 ± 0.2° 2-theta, and/or b) a peak in the DSC record at the temperature of 114°C.

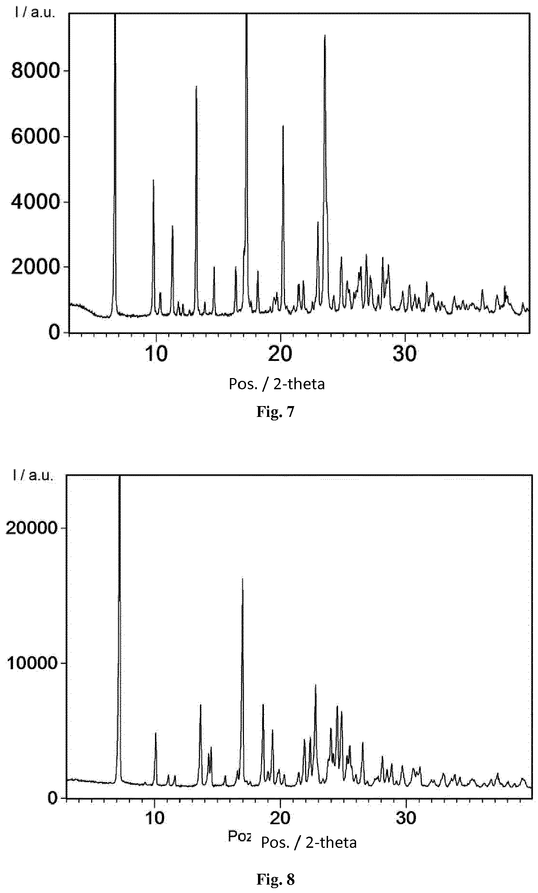

The crystalline form of lesinurad with cyclohexylamine (prepared according to Example 7) is characterized by the reflections presented in Table 5. Table 5 includes reflections whose relative intensity value is higher than 1%. Characteristic diffraction peaks of the crystalline form of lesinurad with cyclohexylamine according to the present invention with the use of CuKα radiation are: 6.7; 9.8; 13.2; 17.2; 20.2 and 23.5 ± 0.2° 2-theta. The X-ray powder pattern is shown in Fig. 7.

**Table 5**

| Position [°2Th.] | Interplanar spacing [Å] [Å]=0.1nm | Rel. intensity [%] |
|---|---|---|
| 6.68 | 13.229 | 92.0 |
| 9.77 | 9.045 | 27.2 |
| 10.31 | 8.575 | 4.7 |
| 11.29 | 7.828 | 18.3 |
| 13.20 | 6.702 | 50.3 |
| 14.62 | 6.052 | 10.8 |
| 16.37 | 5.410 | 10.1 |
| 17.21 | 5.148 | 100.0 |
| 18.12 | 4.891 | 9.3 |
| 20.16 | 4.401 | 41.0 |
| 21.42 | 4.144 | 6.6 |
| 21.79 | 4.076 | 7.3 |
| 22.94 | 3.873 | 20.1 |
| 23.52 | 3.780 | 58.5 |
| 24.83 | 3.583 | 12.0 |
| 25.31 | 3.516 | 6.4 |
| 26.40 | 3.373 | 8.0 |
| 26.83 | 3.321 | 11.4 |
| 27.17 | 3.279 | 7.2 |
| 28.15 | 3.167 | 12.6 |
| 28.64 | 3.115 | 9.7 |
| 29.75 | 3.000 | 3.8 |
| 30.28 | 2.949 | 4.7 |
| 30.76 | 2.905 | 3.6 |
| 31.06 | 2.877 | 3.0 |
| 31.68 | 2.822 | 6.7 |
| 32.18 | 2.780 | 4.0 |
| 36.15 | 2.483 | 5.4 |
| 37.97 | 2.368 | 5.7 |

Differential scanning calorimetry (DSC) was applied to measure the melting point of the crystalline form of lesinurad with cyclohexylamine of 114°C.

In another aspect, the invention relates to a method of preparation of solid forms of lesinurad with an alkylamine that comprises at least the following steps:
a) dissolving or suspending lesinurad and the alkylamine in a solvent selected from a group that consists of alcohols, ketones, esters, ethers, amides, nitriles, organic acids, aliphatic or aromatic hydrocarbons, chlorinated hydrocarbons, water or their mixtures, and
b) removing the solvent.
In a preferred embodiment, the solvent is selected from a group that consists of aliphatic C1-C4 alcohols, ketones, esters, nitriles, water or their mixtures, especially preferably acetone, ethyl acetate, acetonitrile, methanol, ethanol, ethyl methyl ketone, water or their mixtures. In the most preferred embodiment, the solvent is acetone or ethyl methyl ketone.

Before the removal of the solvent, the solution or suspension of lesinurad is preferably incubated for 1 to 24 hours at a temperature from -30°C to the boiling point of the used solvent, more preferably at a temperature from 0 to 130°C, even more preferably at a temperature from 20 to 85°C and most preferably at a temperature from 60 to 80°C. The final solid form is precipitated or crystallized from a solvent, which is preferably removed by filtration, centrifugation or evaporation, either freely or at an elevated temperature, e.g. 40 to 80°C, and/or at a reduced pressure, e.g. 10 kPa (100 mbar).

In a further aspect, this invention provides a solid form of lesinurad with urea that is particularly characterized by high purity. During crystallization of the solid form of lesinurad with urea, chemical impurities originating from the preparation process are removed. These impurities may be residues of the starting compounds or the solvents used in the reactions as well as impurities produced due to insufficient selectivity of the chemical reactions and due to chemical decomposition of the products or intermediates. The said impurities are preferably concentrated in the used solvent, the solid form of lesinurad being separated for example by filtration or centrifugation. The solid form of lesinurad with urea is preferably a cocrystal.

The solid form of lesinurad with urea is particularly suitable as a final, pharmaceutically acceptable product. Thus, this solid form can be used for purification and directly for formulation of a drug form. This makes the entire process of production of the drug form considerably faster and cheaper in comparison to the habitual purification using one solid form of the active ingredient (herein lesinurad) and preparation of the drug form using another solid form of the active ingredient. Urea is the final metabolite of the metabolism of nitrogen, whose normal concentration in blood plasma is 2.5 to 7.5 mmol/l. Urea is removed from the blood by the kidneys and then it is excreted in urine. A small quantity of urea is excreted together with salt by perspiration. Thus, urea is an appropriate pharmaceutically acceptable solid form component.

Another advantageous property of the solid form of lesinurad with urea is its dissolution rate, which is similar to the dissolution rate of free acid, and therefore it can be concluded that when it is used in a drug form, it will be bioequivalent to lesinurad free acid.

Lesinurad and urea can be present in the solid form preferably in the molar ratio of from 1:2 to 2:1, more preferably from 1:1.5 to 1.5:1, most preferably 1:1.

Solid forms of lesinurad with urea having various internal arrangement (polymorphism) with different physicochemical properties were prepared by the inventors within the framework of the present invention. Therefore, the invention also includes individual polymorphic forms of the solid form of lesinurad with urea and their mixtures.

In one embodiment, the solid form of lesinurad with urea is in crystalline form I, which exhibits a) characteristic reflections in the X-ray powder pattern: 7.2; 16.9; 22.8 and 26.5 ± 0.2° 2-theta CuKα, and/or b) a differential scanning calorimetric curve with the melting point at the temperature of 160 ± 2°C. Form I preferably exhibits the following reflections in the X-ray powder pattern: 7.2; 10.1; 13.6; 16.9; 18.6; 22.8; 24.5; 26.5 and 28.1 ± 0.2° 2-theta CuKα. Most preferably, form I exhibits the reflections shown in Table 7, in Example 9, or the X-ray powder pattern shown in Fig. 8.

In another embodiment, the solid form of lesinurad with urea is in crystalline form II, which exhibits a) characteristic reflections in the X-ray powder pattern: 7.3; 16.2; 20.9, 23.6, 29.1 ± 0.2° 2-theta CuKα, and/or b) a differential scanning calorimetric curve with the melting point at the temperature of 147 ± 2°C. Form II preferably exhibits the following reflections in the X-ray powder pattern: 7.3; 8.2; 13.0; 16.2; 20.9; 23.2; 23.6; 24.5; 29.1 and 31.2 ± 0.2° 2-theta CuKα. Most preferably, form II exhibits the reflections shown in Table 8 in Example 10, or the X-ray powder pattern shown in Fig. 9.

The invention also comprises a method of preparation of the solid form of lesinurad with urea that comprises the steps of: a) dissolving or suspending lesinurad and urea in a solvent, b) crystallization and c) removing the solvent. The solvent is preferably selected from a group that consists of C1-C6 alcohols, C3-C6 ketones, C2-C6 esters, C2-C6 ethers, C2-C6 amides, C2-C6 nitriles, organic acids, aliphatic or aromatic hydrocarbons, halogenated hydrocarbons, water or their mixtures. Examples of especially suitable alcohols are methanol, ethanol or isopropyl alcohol. Examples of suitable ketones are acetone and ethyl methyl ketone. An example of suitable esters is ethyl acetate. An example of suitable ethers is tetrahydrofuran. An example of suitable nitriles is acetonitrile. An example of suitable organic acids is acetic acid. An example of suitable aliphatic hydrocarbons is heptane. An example of suitable aromatic hydrocarbons is toluene. An example of halogenated hydrocarbons is dichloromethane.

The solution or suspension of lesinurad with urea is preferably incubated for 1 to 24 hours at a temperature from -30°C to the boiling point of the used solvent, more preferably at a temperature from 0 to 130°C, even more preferably at a temperature from 20 to 85°C and most preferably at a temperature from 40 to 70°C. The resulting solid form is crystallized from the solvent, which is preferably removed by filtration. Alternatively, the final crystalline substance is dried at a reduced pressure in the temperature range of 30°C to 60°C.

In another aspect, the invention relates to novel pharmaceutically acceptable solid forms of lesinurad free acid. These solid forms can be both anhydrous and non-solvated, and in the form of hydrates/solvates of the respective solvents.

In the context of the present invention, the term "solid form of lesinurad free acid" refers to a crystalline or amorphous form of lesinurad in the free acid form, or possibly its hydrates or solvates.

In one embodiment, the solid form of lesinurad free acid is a crystalline form that exhibits at least one of the following properties: a) characteristic reflections in the X-ray powder pattern with the use of CuKα radiation: 7.7; 11.5; 16.9; 19.9 and 26.8° ± 0,2 °2-theta, and/or b) a differential scanning calorimetric curve with the melting point at the temperature of 76°C.

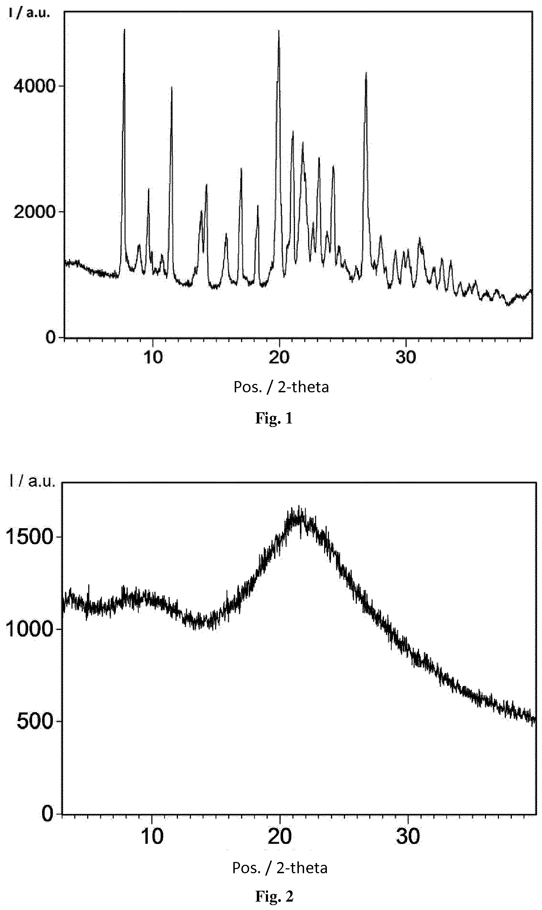

The crystalline form of lesinurad (prepared by the inventors according to Example 1) is characterized by the reflections presented in Table 6. Table 6 includes reflections whose relative intensity value is higher than 1%. Characteristic diffraction peaks of the crystalline form of lesinurad according to the present invention with the use of CuKα radiation are: 7.7; 11.5; 16.9; 19.9 and 26.8° ± 0.2° 2-theta. Other diffraction peaks with the use of CuKα radiation are: 9.6; 14.2; 23.1 and 24.3 ± 0.2 °2-theta. The X-ray powder pattern is shown in Fig. 1.

**Table 6**

| Position [°2Th.] | Interplanar spacing [Å] [Å]=0.1nm | Rel. intensity [%] |
|---|---|---|
| 7.67 | 11.517 | 100.0 |
| 8.87 | 9.967 | 11.5 |
| 9.61 | 9.191 | 33.5 |
| 10.70 | 8.264 | 9.1 |
| 11.47 | 7.706 | 81.2 |
| 13.73 | 6.444 | 35.7 |
| 14.15 | 6.253 | 41.6 |
| 15.73 | 5.629 | 22.2 |
| 16.90 | 5.242 | 50.3 |
| 18.26 | 4.853 | 35.6 |
| 19.94 | 4.450 | 92.7 |
| 21.07 | 4.213 | 47.8 |
| 21.73 | 4.087 | 41.1 |
| 22.62 | 3.927 | 17.7 |
| 23.08 | 3.851 | 52.1 |
| 23.77 | 3.740 | 16.9 |
| 24.25 | 3.667 | 49.3 |
| 24.66 | 3.608 | 12.5 |
| 25.15 | 3.537 | 7.6 |
| 26.76 | 3.329 | 90.0 |
| 27.94 | 3.190 | 18.5 |
| 29.11 | 3.065 | 15.1 |
| 29.75 | 3.001 | 14.2 |
| 30.16 | 2.961 | 17.9 |
| 30.99 | 2.884 | 18.0 |
| 31.30 | 2.855 | 17.5 |
| 32.11 | 2.785 | 11.6 |
| 32.80 | 2.728 | 15.6 |
| 33.49 | 2.674 | 14.9 |
| 34.25 | 2.616 | 6.3 |

Differential scanning calorimetry (DSC) was applied to measure the melting point of the crystalline form of lesinurad free acid of 76°C.

In another aspect, the invention relates to a method of preparation of the said crystalline form of lesinurad free acid that comprises at least the following steps:
a) dissolving or suspending lesinurad free acid in a solvent selected from a group that consists of aliphatic C1-C4 alcohols and their mixtures, and
b) crystallization from a solvent.
In a preferred embodiment of the invention, the solvent is methanol.

In another embodiment of the invention, the solid form of lesinurad free acid is an amorphous form that exhibits at least one of the following properties: a) characteristic amorphous halo in the region of 21.5 ± 0.5° 2-theta in the X-ray powder pattern with the use of CuKα radiation (also see Fig. 2) and/or b) a differential scanning calorimetric curve with the glass transition temperature Tg > 55°C.

In another aspect, the invention relates to a method of preparation of the said amorphous form of lesinurad free acid that comprises at least the following steps:
a) dissolving or suspending lesinurad free acid in a solvent selected from a group that consists of aliphatic C1-C4 alcohols, ketones, ethers, esters, nitriles, water or their mixtures, preferably acetone, ethyl acetate, acetonitrile, methanol, ethanol, water or their mixture, and
b) quick removal of the solvent, e.g. by spray drying or evaporation on a rotary vacuum evaporator.
In a preferred embodiment of the invention, the solvent is a mixture of methanol and tetrahydrofuran, e.g. at the ratio of 3 : 1.

Another object of the invention is a pharmaceutical composition comprising the solid form of lesinurad with at least one amino group-containing organic base.

In particular, the pharmaceutical componsition comprises the solid form of lesinurad with urea.

The pharmaceutical composition is preferably in a form suitable or intended for oral administration. The pharmaceutical composition may be prepared in any solid drug form, e.g. in the form of tablets, capsules, powder, pellets, or granules. A preferred drug form is a tablet or coated tablet.

The pharmaceutical composition can be advantageously prepared in such a manner that the solid form of lesinurad with urea is mixed with pharmaceutically acceptable auxiliary substances and subjected to tabletting, which is optionally followed by coating.

The composition further typically comprises one or more pharmaceutically acceptable excipients that serve especially as fillers, binders, lubricants, surfactants, disintegrants, dyes, solvents, antimicrobial substances or as taste and smell correctors.

The pharmaceutical composition preferably contains a quantity of the solid form of lesinurad with urea corresponding to the strength of 100 to 300 mg of lesinurad free acid, preferably corresponding to the strength of 200 mg of lesinurad free acid.

Another object of the invention is the solid form of lesinurad with at least one amino group-containing organic base , in particular the solid form of lesinurad with urea, for use as a drug, especially for use for treating hyperuricemia.

### Brief description of the Drawings

Fig. 1 represents the X-ray powder pattern of the crystalline form of lesinurad free acid
Fig. 2 represents the X-ray powder pattern of the amorphous form of lesinurad free acid
Fig. 3 represents the X-ray powder pattern of the crystalline form of lesinurad with tromethamine (crystalline form I)
Fig. 4 represents the X-ray powder pattern of the crystalline form of lesinurad with tromethamine (crystalline form II)
Fig. 5 represents the X-ray powder pattern of the crystalline form of lesinurad with diethylamine
Fig. 6 represents the X-ray powder pattern of the crystalline form of lesinurad with tert-butylamine
Fig. 7 represents the X-ray powder pattern of lesinurad with cyclohexylamine
Fig. 8 represents the X-ray powder pattern of form I of the solid form of lesinurad with urea.
Fig. 9 represents the X-ray powder pattern of form II of the solid form of lesinurad with urea.
Fig. 10 represents the X-ray powder pattern of purified crystalline form of lesinurad with tromethamine (crystalline form II).

### Examples of carrying out the Invention

### Methods:

### X-ray powder diffraction

The diffractograms were obtained using an X'PERT PRO MPD PANalytical powder diffractometer, used radiation CuKα (λ=1.542 Å), excitation voltage: 45 kV, anode current: 40 mA, measured range: 2 - 40° 2θ, increment: 0.01° 2θ at the dwell time at a reflection of 0.5 s, the measurement was carried out with a flat sample with the area/thickness of 10/0.5 mm. For the correction of the primary array 0.02 rad Soller slits, a 10mm mask and a 1/4° fixed anti-dispersion slit were used. The irradiated area of the sample is 10 mm, programmable divergence slits were used. For the correction of the secondary array 0.02 rad Soller slits and a 5.0 mm anti-dispersion slit were used.

### Differential Scanning Calorimetry (DSC)

The records of the solid crystalline forms of lesinurad were measured with the use of a DSC Pyris 1 device by Perkin Elmer. The sample charge in a standard Al pot was between 2.5-3.0 mg and the heating rate was 10°C/min. The temperature program that was used consists of 1 min of stabilization at the temperature of 0°C and then of heating up to 300°C at the heating rate of 10°C/min. As the carrier gas 4.0 N₂ was used at the flow of 20 ml/min.

The record of the amorphous form of lesinurad was measured using a Discovery DSC device made by TA Instruments. The sample charge in a standard Al pot (40 µL) was between 4 and 5 mg and the heating rate was 5°C/min. The temperature program that was used consists of 1 min of stabilization at the temperature of 0°C and then of heating up to 250°C at the heating rate of 5°C/min (amplitude = 0.8°C and period = 60 s). As the carrier gas 5.0 N₂ was used at the flow of 50 ml/min.

### Spray drying

The removal of the solvent with the use of spray drying was carried out by means of a BUCHI B290 laboratory spray drier. Carrier gas flow 40 m³/h, inlet temperature 120°C, outlet temperature 95°C, solution dosage rate 6%, aspirator 95%, condensation loop temperature 0°C.

### Determining the dissolution rate using the true dissolution method

The dissolution rate was measured using a Sirius inForm dissolution device. Disks for true dissolution with the surface area available for dissolution of 0.071 cm² (d = 0.3 cm) were prepared using a Specac press. The pressing time was 2x2 minutes at the pressing pressure of 100 kg. The dissolution was carried out in 40 ml of a solution at pH 6.8 (38 ml of 150mM NaCl + 2 ml of 150mM NaCl, 100mM CH₃COONa, 100mM NaH₂PO₄), at the constant speed of 100 rpm. Samples were drawn at 5-second intervals and the concentration of dissolved lesinurad was determined by means of an integrated UV/Vis spectrophotometric probe in the wavelength range of 230-400 nm.

### Reference Example 1

Crystalline form 2 of lesinurad free acid was prepared in accordance with the process disclosed in the patent application WO 2012/092395. Differential scanning calorimetry (DSC) was applied to measure the melting point of 172°C.

### Example 1

### Preparation of a crystalline form of lesinurad free acid.

0.999 g of lesinurad free acid, prepared according to Reference Example 1, was dissolved under reflux conditions with a return cooler in 4 ml of methanol. This solution was filtered through a PTFE filter (pore size 0.2 µm) in a syringe into a cooled 25 ml flask, which was left to cool in the ice bath. The obtained suspension was left to be stirred for 30 min in the ice bath. Then, the remaining methanol was evaporated on a rotary vacuum evaporator at 25°C and the pressure of 4,500 Pa. The solid fraction was further dried in a vacuum drier at 40°C and the pressure of 10,000 Pa for 8 hours.

The X-ray powder pattern is shown in Fig. 1. The melting point of the crystalline form of lesinurad, determined by means of DSC, is 76°C.

The crystalline form of lesinurad was prepared by abrupt cooling of the methanolic solution. This solid form exhibits a lower melting point and thus also higher solubility and bioavailability than the known thermodynamically most stable polymorph of lesinurad (form 2). The prepared product can be further used for the preparation of a solid drug form and/or other solid forms of lesinurad.

### Example 2

### Preparation of an amorphous form of lesinurad free acid.

5 g of lesinurad free acid, prepared according to Reference Example 1, was dissolved in 200 ml of the methanol : tetrahydrofuran 3:1 mixture. The solvent was evaporated with the use of a BUCHI B290 spray drier.

The X-ray powder pattern is shown in Fig. 2. The glass transition temperature of the amorphous solid form of lesinurad free acid, determined by means of DSC, is 55°C.

### Example 3

### Crystalline form of lesinurad with tromethamine (form I)

6.3 g of lesinurad free acid, prepared according to Reference Example 1, was dissolved at 40°C in ethyl methyl ketone (40 ml) and tromethamine (1.9 g) in water (4 ml) was added to the obtained solution by dripping. The suspension was heated for 4 h at 40°C and then cooled at 25°C for 4 h. After suctioning and drying in vacuum, the amount of 6.6 g (80%) of the product was obtained in a crystalline form.

The X-ray powder pattern is shown in Fig. 3. The melting point of the crystalline form of lesinurad with tromethamine (form I), determined by means of DSC, is 169°C. The ratio of lesinurad and tromethamine in crystalline form I was 1:1.

The prepared crystalline form I was subjected to tests from the point of view of chemical and polymorphic stability. After 3 months at 40°C / 75% humidity, form I did not show any signs of chemical degradation or transition to a different polymorphic form. The same results were also obtained after 3 days at 80°C.

### Example 4

### Crystalline form of lesinurad with tromethamine (form II)

250 mg of lesinurad free acid, prepared according to Reference Example 1, was dissolved in 3 ml of acetone under reflux conditions with a return cooler. 73 mg of tromethamine was added to this solution and the solution was left to cool down to the laboratory temperature under continuous stirring.

The solvent was left to freely evaporate and the solid fraction was dried in a vacuum drier at 40°C and the pressure of 4,000 Pa for 8 hours.

The X-ray powder pattern is shown in Fig. 4. The melting point of the crystalline form of lesinurad with tromethamine (form II), determined by means of DSC, is 177°C. The ratio of lesinurad and tromethamine in crystalline form II was 1:1.

### Example 5

### Crystalline form of lesinurad with diethylamine

5.85 g of lesinurad free acid, prepared according to Reference Example 1, was dissolved in 50 ml acetone at a temperature of approx. 55°C. Diethylamine (1.5 ml) was added to this solution and the mixture was left to be stirred and slowly cool down to the laboratory temperature. The obtained suspension was left to be stirred for 1 hour at the laboratory temperature. The solid fraction was separated by filtration, washed with acetone and further dried in a vacuum drier at 60°C and the pressure of 10,000 Pa for 8 hours.

The X-ray powder pattern is shown in Fig. 5. The melting point of the crystalline form of lesinurad with diethylamine, determined by means of DSC, is 117°C. The ratio of lesinurad and diethylamine in the crystalline form was 1:1.

### Example 6

### Crystalline form of lesinurad with tert-butylamine

0.5 g of lesinurad free acid, prepared according to Reference Example 1, was dissolved in acetone (3 ml) at 25°C. After addition of tert-butylamine (0.18 g) and acetic acid (0.18 g), the product crystallized, providing the amount of 0.45 g.

The X-ray powder pattern is shown in Fig. 6. The melting point of the crystalline form of lesinurad with tert-butylamine, determined by means of DSC, is 157°C. The ratio of lesinurad and tert-butylamine in the crystalline form was 1:1.

### Example 7

### Crystalline form of lesinurad with cyclohexylamine

0.5 g of lesinurad free acid, prepared according to Reference Example 1, was dissolved in acetone (5 ml) at 25°C. After addition of cyclohexylamine (0.1 g) and acetic acid (0.2 g), the product crystallized, providing the amount of 0.38 g.

The X-ray powder pattern is shown in Fig. 7. The melting point of the crystalline form of lesinurad with cyclohexylamine, determined by means of DSC, is 114°C. The ratio of lesinurad and cyclohexylamine in the crystalline form was 1:1.

### Example 8

### Lesinurad free acid

Tetrahydrofuran (25 ml) and *N*,*O*-bis(trimethylsilyl)acetamide (3.1 ml, 1.25 equivalents) were added to 2-(4-(1-cyclopropylnaphtalen-4-yl)-4*H*-1,2,4-triazol-3-ylthio)acetic acid (**2**, 3.25 g). The mixture was stirred at 25°C for 5 to 10 minutes and monitored by means of HPLC. Then, N-bromosuccinimide (2.67 g, 1.5 equivalents) was added, the obtained solution was stirred, heated up to the selected temperature (50°C) and the course of the reaction (conversion of the input compound) was monitored by means of HPLC. The conversion of the input compound was approx. 100%, 90 minutes after the addition of N-bromosuccinimide. 4-methyl-2-pentanone (100 ml) was added to the reaction mixture, THF was evaporated under vacuum. The obtained solution was washed with water (50 ml), a solution of 10% sodium thiosulfate (25 ml), water again (50 ml) and dried over sodium sulfate. After concentration and crystallization from acetone (30 ml), lesinurad free acid (3 g, 74%) was obtained, having the HPLC purity of 97%, which was identified as form 2 in accordance with WO2012092395.

### Example 9

### Solid form of lesinurad with urea (1:1) - form I

6.7 g of lesinurad free acid, having the HPLC purity of 97%, was stirred up in ethyl acetate (67 ml) and urea (1 g), which was dissolved in a hot state in methanol (6 ml), was added to the obtained suspension. The obtained solution was filtered at 50°C while the product quickly crystallized in the filtrate. The resulting suspension was stirred for 2 h at 50°C and then overnight (12 h) at the laboratory temperature. After filtration, 6.65 g of the product with the HPLC purity of 99.7% was obtained. ¹H NMR (500 MHz, DMSO) δ 8.58 (d, *J*=8.5 Hz, 1H), 7.74 (t, *J*=7.7 Hz, 1H), 7.65 (t, *J*=7.7 Hz, 1H), 7.63 (d, *J*=7.6 Hz, 1H), 7.44 (d, *J*=7.6 Hz, 1H), 7.15 (d, *J*=8.4 Hz, 1H), 5.42 (s, 4H, urea), 4.015 and 3.975 (2d, *J*=16.4, 2H), 2.55 (m, 1H), 1.15 (m, 2H), 0.87 (m, 2H).

The X-ray powder pattern is shown in Fig. 8. Reflections whose relative intensity values are higher than 2% are presented in Table 7. Characteristic diffraction peaks of the crystalline form of the solid form of lesinurad with urea according to the present invention with the use of CuKα radiation are: 7.2; 16.9; 22.8 and 26.5 ± 0.2° 2-theta. More diffractions peaks are found in the positions: 10.1; 13.6; 18.6; 24.5 and 28.1 ± 0,2° 2-theta. Differential scanning calorimetry (DSC) was applied to measure the melting point of the crystalline form I of the solid form of lesinurad with urea of 160 ± 2°C.

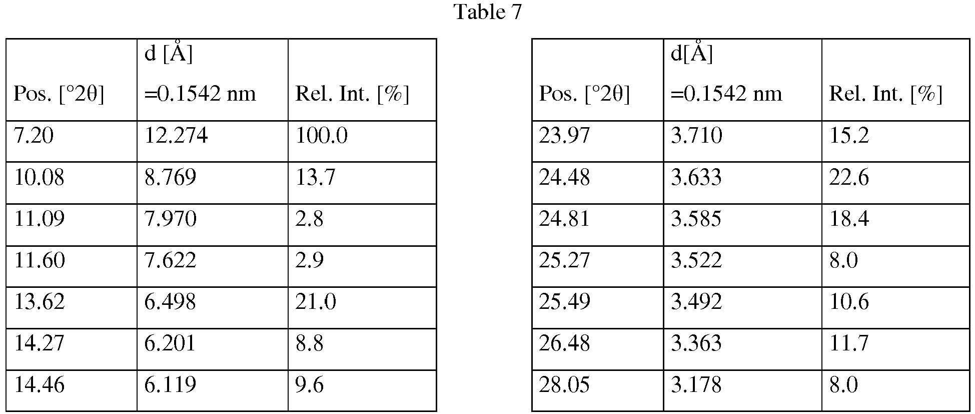

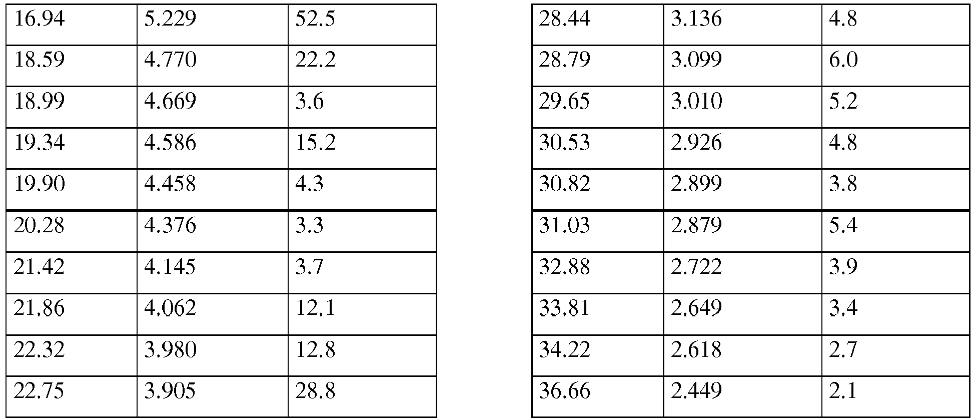

### Example 10

### Solid form of lesinurad with urea - form II

80 mg of the solid form of lesinurad with urea obtained according to Example 9 was suspended in a mixture of ethyl acetate : methanol (1:1, 0.3 ml) and stirred for a week at the temperature of 50°C. After evaporation, form II of the solid form of lesinurad with urea (1:1) was obtained.

The X-ray powder pattern is shown in Fig. 9. Reflections whose relative intensity values are higher than 2% are presented in Table 8. Characteristic diffraction peaks of the crystalline form of the solid form of lesinurad with urea according to the present invention with the use of CuKα radiation are: 7.3; 16.2; 20.9; 23.6 and 29.1 ± 0,2° 2-theta. More diffractions peaks are found in the positions: 8.2; 13.0; 23.2; 24.5 and 31.2 ± 0,2° 2-theta. Differential scanning calorimetry (DSC) was applied to measure the melting point of the crystalline form II of the solid form of lesinurad with urea of 147 ± 2°C.

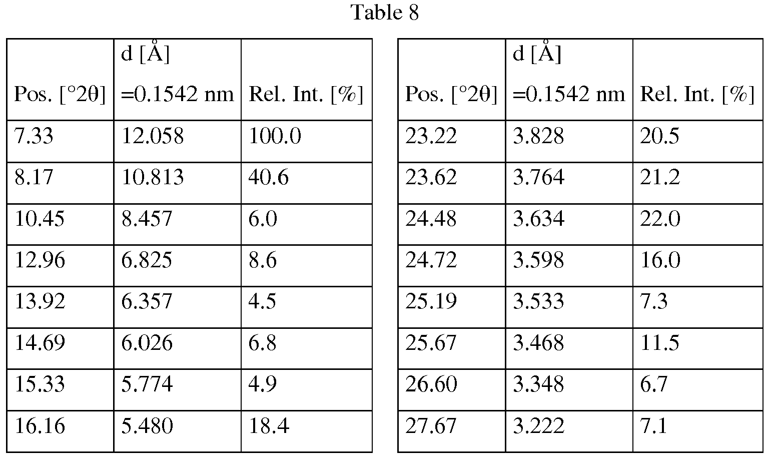

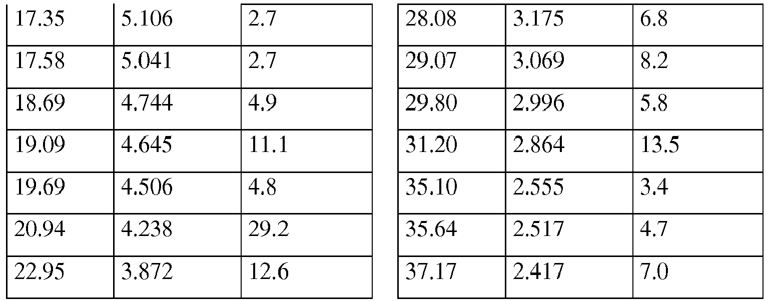

### Example 11

Alternative method of preparation of form II of the solid form with urea 80 mg of the solid form of lesinurad with urea obtained according to Example 9 was suspended in ethyl methyl ketone (0.3 ml) and stirred for a week at 50°C. After evaporation, form II of the solid form with urea was obtained that was identical to the form described in Example 10.

### Example 12

### Crystalline form of lesinurad with tromethamine (purified form II)

The crystalline form of lesinurad with tromethamine obtained in Example 11 (100 mg) is maintained at 40 °C for 24 hours in 1 ml acetone. After vacuum filtration, pure form II is obtained (the XRD pattern is shown in Fig. 10).

### Example 13

### Determining the dissolution rate

Disks for measuring true dissolution (d = 3 mm) were produced from the prepared solid forms of lesinurad, i.e. free acid form 2 (reference form), tromethamine salt form I and diethylamine salt, and solid form of lesinurad with urea (form I), with the use of a press. The dissolution was carried out in 40 ml of an acetate-phosphate buffer at pH 6,8 (150mM NaCl, 5mM CH₃COONa, 5mM NaH₂PO₄), at the constant stirring speed of 100 rpm. Samples were drawn at 5-second intervals and the concentration of dissolved lesinurad was determined by means of a UV/Vis spectrophotometer. The dissolution rate of lesinurad free acid (form 2) was 0.42 mg/min/cm², the dissolution rate of lesinurad with tromethamine (form I) measured at identical conditions was 7.44 mg/min/cm², the dissolution rate of lesinurad with dimethylamine was 21.58 mg/min/cm², and the dissolution rate of the solid form of lesinurad with urea (form I) was 0.87 mg/min/cm².

## Claims

1. A solid form of lesinurad with an amino group-containing organic base of formula
R¹-NH-R²,
wherein the R¹ and R² groups are independently selected from the group consisting of -C(=O)-NH₂, hydrogen, a linear or branched C1-C6 alkyl or C1-C6 cycloalkyl wherein the alkyl or cycloalkyl are optionally substituted by at least one hydroxy group,
provided that at least one of the R¹ and R² groups is not hydrogen.

2. The solid form according to claim 1, wherein
- R¹ is hydrogen or methyl and R² is -C(=O)-NH₂; or
- R¹ and R² groups are independently selected from the group consisting of hydrogen or a linear or branched C1-C6 alkyl or C1-C6 cycloalkyl, wherein the alkyl or cycloalkyl are substituted by at least one hydroxy group, provided that at least one of the R¹ and R² groups is not hydrogen.

3. The solid form according to claim 1, wherein the amino group-containing organic base is selected from urea, tromethamine, diethylamine, cyclohexylamine, or tert-butylamine; preferably selected from urea and tromethamine.

4. The solid form according to claim 3, wherein the amino group-containing organic base is tromethamine, and wherein the solid form exhibits
a1) characteristic reflections in the X-ray powder pattern: 10.5; 14.1; 17.4; 21.6; 23.9 and 25.7 ± 0.2° 2-theta, and/or
b1) a differential scanning calorimetric curve with the melting point at the temperature of 169°C;
or
a2) characteristic reflections in the X-ray powder pattern: 9.2; 10.3; 14.3; 17.2; 18.9; 22.8 and 24.1 ± 0.2° 2-theta, and/or
b2) a differential scanning calorimetric curve with the melting point at the temperature of 177°C.

5. The solid form according to claim 3, selected from:
- the solid form wherein the amino group-containing organic base is diethylamine, and wherein the solid form exhibits
a) characteristic reflections in the X-ray powder pattern: 7.3; 10.1; 15.7; 21.7; 23.0 and 25.1 ± 0.2° 2-theta, and/or
b) a differential scanning calorimetric curve with the melting point at the temperature of 117°C;
- the solid form wherein the amino group-containing organic base is tert-butylamine, and wherein the solid form exhibits
a) characteristic reflections in the X-ray powder pattern: 10.1; 13.1; 17.8; 20.1; 22.4 and 25.8 ± 0.2° 2-theta, and/or
b) a differential scanning calorimetric curve with the melting point at the temperature of 157°C;
- the solid form wherein the amino group-containing organic base is cyclohexylamine, and wherein the solid form exhibits
a) characteristic reflections in the X-ray powder pattern: 6.7; 9.8; 13.2; 17.2; 20.2 and 23.5 ± 0.2° 2-theta, and/or
b) a differential scanning calorimetric curve with the melting point at the temperature of 114°C.

6. The solid form according to claim 3, wherein the solid form is a solid form of lesinurad with urea.

7. The solid form according to claim 6, wherein the solid form of lesinurad with urea exhibits
a1) characteristic reflections in the X-ray powder pattern: 7.2; 16.9; 22.8 and 26.5 ± 0.2° 2-theta CuKα, and/or
b1) a differential scanning calorimetric curve with the melting point at the temperature of 160 ± 2°C;
or
a2) characteristic reflections in the X-ray powder pattern: 7.3; 16.2; 20.9; 23.6 and 29.1 ± 0.2° 2-theta CuKα and/or
b2) a differential scanning calorimetric curve with the melting point at the temperature of 147 ± 2°C.

8. The solid form according to claim 7, wherein the solid form of lesinurad with urea exhibits
- the following reflections in the X-ray powder pattern: 7.2; 10.1; 13.6; 16.9; 18.6; 22.8; 24.5; 26.5 and 28,1 ± 0.2° 2-theta CuKα;
or
- the following reflections in the X-ray powder pattern: 7.3; 8.2; 13.0; 16.2; 20.9; 23.2; 23.6; 24.5; 29.1 and 31.2 ± 0.2° 2-theta CuKα.

9. A solid form of lesinurad free acid, exhibiting
a1) characteristic reflections in the X-ray powder pattern with the use of CuKα radiation: 7.7; 11.5; 16.9; 19.9; 26.8 and 0.2 ± 2° 2-theta, and/or
b1) a differential scanning calorimetric curve with the melting point at the temperature of 76°C;
or
a2) a characteristic amorphous halo in the region of 21.5 ± 0.5° 2-theta in the X-ray powder pattern with the use of CuKα radiation, and/or
b2) a differential scanning calorimetric curve with the glass transition temperature Tg > 55°C.

10. A method for preparing the solid form of lesinurad with an amino group-containing organic base as defined in claim 1, **characterized in that** it comprises the steps of
a) dissolving or suspending lesinurad and an amino group-containing organic base in a solvent selected from a group consisting of alcohols, ketones, esters, ethers, amides, nitriles, organic acids, aliphatic or aromatic hydrocarbons, chlorinated hydrocarbons, water or their mixtures, and
b) removing the solvent.

11. The method according to claim 10, **characterized in that** the solvent is selected from a group consisting of aliphatic C1-C4 alcohols, ketones, esters, nitriles, water or their mixtures, preferably the solvent is selected from acetone, ethyl acetate, acetonitrile, methanol, ethanol, ethyl methyl ketone, water or their mixtures, more preferably the solvent is acetone or ethyl methyl ketone.

12. The method according to claim 10, **characterized in that** the solid form is the solid form of lesinurad with urea and **in that** it comprises the following steps:
a) dissolving or suspending lesinurad and urea in a solvent, preferably selected from a group consisting of C1-C6 alcohols, C3-C6 ketones, C2-C6 esters, C2-C6 ethers, C2-C6 amides, C2-C6 nitriles, organic acids, aliphatic or aromatic hydrocarbons, halogenated hydrocarbons, water or their mixtures,
b) crystallization, and
c) removing the solvent.

13. A pharmaceutical composition, **characterized in that** it comprises the solid form of lesinurad with at least one amino group-containing organic base as defined in claim 1, preferably it comprises the solid form of lesinurad with urea; preferably the pharmaceutical composition is in a form for oral administration.

14. The solid form of lesinurad with at least one amino group-containing organic base according to any one of claims 1 to 8 for use as a medicament, preferably for use in a method of treatment of hyperuricemia.

15. The solid form for use according to claim 14, wherein the solid form of lesinurad with at least one amino group-containing organic base is a solid form of lesinurad with urea.
